(19)

Europäisches Patentamt
European Patent Office
Office européen des brevets

(11) **EP 3 219 256 A1**

(12) **EUROPEAN PATENT APPLICATION**

(43) Date of publication:
**20.09.2017 Bulletin 2017/38**

(51) Int Cl.:
*A61B 5/16* (2006.01)        *B60K 35/00* (2006.01)
*B60W 40/08* (2012.01)        *G01C 21/36* (2006.01)

(21) Application number: **16161159.5**

(22) Date of filing: **18.03.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Valeo Comfort and Driving Assistance 94046 Créteil Cedex (FR)**

(72) Inventor: **NAGIUB, Mena 94046 Créteil CEDEX (FR)**

(74) Representative: **Delplanque, Arnaud VALEO Comfort and Driving Assistance 76, rue Auguste Perret Z.I. Europarc 94046 Créteil Cedex (FR)**

(54) **A METHOD FOR DETERMINING A VIGILANCE STATE OF A DRIVER OF A VEHICLE**

(57)    The invention deals with a method for determining a vigilance state of a driver of a vehicle that comprises a steering wheel (1) including at least a first sensor (10) adapted to detect a contact or a pressure exerted by the hand of said driver on said steering wheel, said method including steps of:
- measuring a variable relative to the contact or to the pressure exerted by the hand of the driver on the steering wheel, thanks to said first sensor,
- comparing said variable with a first threshold, and
- deducing from the result of said comparison a first value of the vigilance state of the driver.
    According to the invention, the vehicle comprises at least a second sensor (20) adapted to measure a parameter used to determine a second value of the vigilance state of the driver, and said first threshold is determined on the basis of said second value.

Fig.1

EP 3 219 256 A1

## Description

The present invention relates in general to the analyzing of the vigilance of a driver of a vehicle.

The invention applies more particularly to a method for determining a vigilance state of a driver of a vehicle, which vehicle comprises a steering wheel including at least a first sensor adapted to detect a contact or a pressure exerted by the hand of said driver on said steering wheel, said method including steps of:

- measuring a variable relative to the contact or to the pressure exerted by the hand of the driver on the steering wheel, thanks to said first sensor,
- comparing said variable with a first threshold, and
- deducing from the result of said comparison a first value of the vigilance state of the driver.

### TECHNOLOGICAL BACKGROUND

In recent times, the number of potential distractions for a driver has increased. With the advent of portable electronics (e.g., mobile phones, mp3 players, etc.), there are a number of electronic distractions that encourage or tempt drivers to remove their hands from the steering wheel.

Moreover, a driver who drives for a long time or during night is subjected to fatigue and drowsiness.

That is why it is desirable to provide a system for detecting such a distraction or drowsiness state in order to avoid any accident.

A solution to detect distraction state of the driver is known from document US20110246028. This solution consists in using a steering wheel that includes a plurality of pressure sensitive sensors, and a control unit that is able to determine if at least one of the driver's hands is pressing upon a region of the steering wheel for a predetermined amount of time.

A problem of this solution is that, if it is well adapted to a particular profile of drivers (for instance inexperienced drivers), it does not work well for other drivers whose habits are different.

Thus, because this solution does not take into account the driver's behavior, it is not sufficiently reliable.

### OBJECT OF THE INVENTION

The object of the present invention consists in finding a solution that is suitable for detecting a fatigue or drowsiness state with a better reliability.

This invention consists in a method as disclosed in introduction wherein, the vehicle comprising at least a second sensor adapted to measure a parameter able to determine a second value of the vigilance state of the driver, said first threshold is determined on the basis of said second value.

Thanks to the invention, the detection of a lack of vigilance is made with two different sensors. The second sensor is used to set the first threshold to a value that varies according to the profile of the driver. Consequently, when the first sensor is well calibrated, the detection of a lack of vigilance with the first sensor becomes more reliable.

Thus, when the second sensor is no longer able to determine the vigilance level of the driver, for instance because the luminosity decreases, the determination of this vigilance level with the first sensor remains reliable.

Other advantageous features of the invention are the following ones:

- the determination of said first threshold is implemented during a learning phase that is prior to an action phase during which said first value is deduced from the result of said comparison;
- the learning phase includes steps of:

    S1) setting said first threshold to a predetermined default value,
    S2) comparing said variable with the default value of the first threshold,
    S3) deducing from the result of step S2) a preliminary value of said vigilance state,
    S4) acquiring the second value of said vigilance state, and
    S5) adjusting the value of said first threshold depending on said preliminary value and said second value;

- the learning phase also includes steps of determining a correctness variable depending on said preliminary value and said second value, and, if said correctness variable is above a correctness level, switching to said action phase, else, repeating steps S1) to S5) ;
- said second sensor being adapted to acquire images of the face of the driver, said first threshold is determined on the basis of the movements of the eyelids of the driver detected on the acquired images;
- the variable measured by said first sensor is a hand pressure exerted by the hand of the driver on the steering wheel, and the first value of said vigilance state is equal to or deduced from a first drowsiness level determined on the basis of the result of the comparison between said variable and said first threshold;
- the variable measured by said first sensor is a gripping force exerted by the driver on the steering wheel, and the first value of said vigilance state is equal to or deduced from a second drowsiness level determined on the basis of the result of the comparison between said variable and said first threshold;
- the first value of said vigilance state is equal to or deduced from the multiplication of said first drowsiness level by said second drowsiness level;
- the variable measured by said first sensor is a contact area between the hand of the driver and the steering wheel, and the first value of said vigilance state is equal to or deduced from a distraction level determined on the basis of the result of the comparison between said variable and said first threshold;

- at said step of deduction, the first value of said vigilance state is reduced if said variable remains below said first threshold during a predetermined amount of time;

- at said step of deduction, the first value of said vigilance state, after it has been reduced, is increased if said variable rises above a second threshold that is distinct from said first threshold; and

- the method comprises a last step of determining a parameter relative to the behavior for said driver, on the basis of said first threshold.

DETAILED DESCRIPTION OF AN EMBODIMENT

The following description with reference to the accompanying drawings, given by way of non-limiting example, makes it clear what the invention consists in and how it can be reduced to practice.

In the accompanying drawings:

- Figure 1 is a schematic view of a steering wheel, a camera and a processing unit suitable for implementing a method for determining a vigilance state of a driver of a vehicle according to the invention,

- Figure 2A is a graphic illustrating the variation over time of a variable measured by a first sensor comprised in the steering wheel of Figure 1, and

- Figure 2B is a graphic illustrating the variation over time of the vigilance state determined thanks to the variable of Figure 2A.

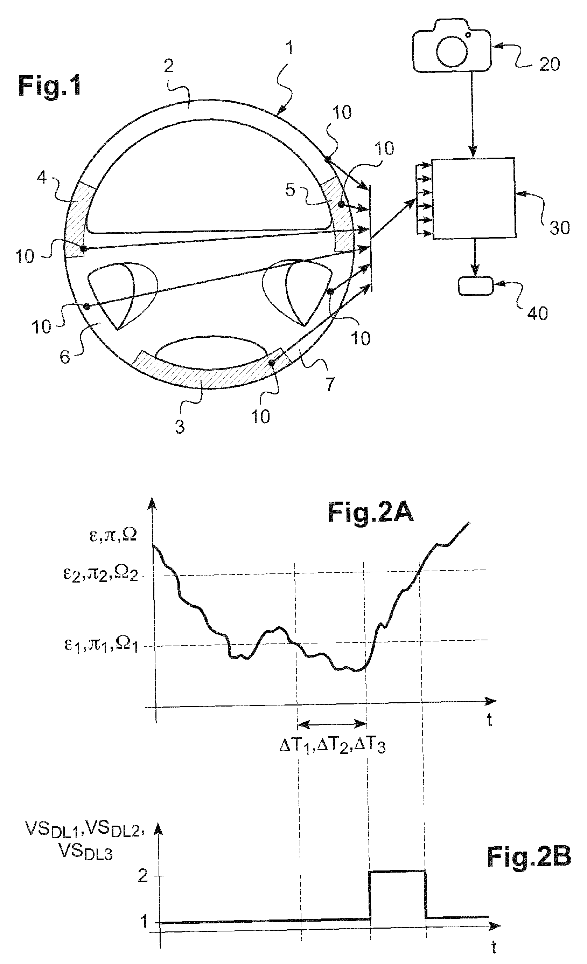

Figure 1 shows parts of a vehicle.

It shows first a steering wheel 1 that includes at least a first sensor 10.

It also shows a second sensor 20 and a processing unit 30 that is connected to the first and second sensors 10, 20 and that is able to determine the vigilance state of the driver of the vehicle, on the basis of the values measured by these sensors 10, 20.

Finally, it shows actuator means 40 that are driven by the processing unit 30 and that are able to make an action when the determined vigilance state is low. Examples of such actions will be described below.

The or each first sensor 10 is adapted to measure a variable that is able to be used to determine the driver's vigilance state VS.

More precisely, the or each first sensor 10 may produce an output signal that represents the magnitude of a variable $\varepsilon$, $\pi$, $\Omega$ relative to the contact or to the pressure exerted by the hands of the driver on the steering wheel 1.

Here, a first variable measured is a hand pressures $\pi$ exerted by the hand of the driver on the steering wheel 1.

A second variable measured is a gripping force $\varepsilon$ exerted by the driver on the steering wheel 1.

A third variable measured is a contact area $\Omega$ between the hand of the driver and the steering wheel 1.

The hand gripping force $\varepsilon$ is defined as the force exerted by the fingers of the driver's hand on the annular gripping part of the steering wheel 1, while the hand pressures $\pi$ is defined as the pressure applied by the palm of the driver's hand (regardless of the fingers gripping force) on the front face of the steering wheel 1.

The contact area $\Omega$ is defined as the steering wheel surface that is covered by the driver's hands. It varies according to the contact strength exerted by the driver's hands on the steering wheel 1. Regarding how hand contact area $\Omega$ may vary, the more the driver is applying a pressure on the steering wheel 1, the more the hand palm of the driver will be in direct contact with the steering wheel 1 (so that the contact area $\Omega$ will increase).

It is expected that when the driver is awake, he or she will apply great hand palm pressures $\pi$ and gripping force $\varepsilon$ on the steering wheel 1 and hence the contact area $\Omega$ should increase. When the driver becomes drowsy, hand palm pressures $\pi$ and gripping force $\varepsilon$ will decrease due to relaxation in the hand muscles and hence the contact area $\Omega$ should decrease.

These variables may be measured by the same sensors. But here, the first and second variables $\varepsilon$, $\pi$ are measured by sensors that are distinct from the sensors used to measure the third variable $\Omega$.

The gripping force and hand pressure sensors could be realized by different ways, for instance thanks to an array of piezoelectric sensors.

The hand contact area sensor could be realized using a capacitive touch sensor film enclosed in the skin of the steering wheel 1.

More specifically, the annular gripping part of the steering wheel 1 is divided into six sections as follows:

- a top part 2,
- a hatched bottom part 3,
- a hatched top left part 4,
- a hatched top right part 5,
- a bottom left part 6, and
- a bottom right part 7.

The hatched parts 3, 4, 5 represent the areas with high potential for a driver hand contact while the other parts 2, 6, 7 represent the areas with low or no potential for a driver hand contact.

Thus, the majority or all of the first sensors 10 are distributed in the hatched parts 3, 4, 5, on the front side of the steering wheel. In a variant, they also could cover the back side of the steering wheel 1 to increase the detection area for the hand contact sensing.

These first sensors 10 are all connected to the processing unit 30 by cables and via analogue to digital converters.

The or each second sensor 20 is adapted to measure the value of a parameter that is able to be used to determine the driver's vigilance state VS.

This second sensor may be realized in different ways.

For instance, this second sensor could be a position sensor, suitable for measuring the steering angle of the steering wheel 1. Indeed, it is known that the variation of the steering angle may indicate if the driver is vigilant or not.

Another example may be a sensor housed in the driver's seat of the vehicle and adapted to measure a parameter relative to the position of the driver. Indeed, it is known that the position of the driver may indicate if the driver is vigilant or not.

In the represented embodiment, the second sensor 20 is adapted to acquire images of the face of the driver. Indeed, such images may be processed to determine the movements of the lids of the driver and to deduce therefrom if the driver is vigilant or not.

Here, this second sensor is a video camera 20 which may be located on the driver's visor, dash-board or other suitable location to enable video monitoring of the driver's eyes. An infra-red lens may be utilized to facilitate reliable night video monitoring capability.

This camera video 20 is connected to the processing unit 30 by cables and via an analogue to digital converter.

Signals from the various sensors 10, 20 may be processed and analyzed by the processing unit 30.

To process and analyze these signals, the processing unit 30 comprises a processor (CPU), a random access memory (RAM), a read only memory (ROM), converters analog-digital, and various input and output interfaces.

In its RAM memory, the processing unit 30 stores in particular thresholds that will be described in detail in the following.

In its ROM memory, the processing unit 30 stores default values of these thresholds.

The ROM memory also includes a video processing algorithm suitable to process the signal received from the video camera 20, which includes an eye recognition software designed to identify eyes in contrast to other parts of the drivers face. The analysis processed by the algorithm includes determining the area of the eye's opening and correlating the eye's opening area to previous similar measurements. In this way eye processing can determine whether the driver's eyes are remaining open as would be expected in an alert state or whether the current eye opening of the driver is relatively less (when compared to earlier eye opening measurements). Rates or degrees of eye closure are able to be detected and continually monitored in this manner.

Through its input interfaces, the processing unit 30 is adapted to receive the signals from the first sensors 10 and from the video camera 20.

Thanks to its processor, the processing unit 30 is adapted to generate, as a function of the received signals, output signals.

Finally, through its output interfaces, the processing unit 30 is adapted to transmit the output signals to the actuator means 40.

These actuator means 40 are arranged to intervene in the control of the vehicle and/or to alert the driver of the vehicle and/or to alert the drivers of other vehicles, when the vigilance state of the driver is low.

Vehicle control intervention may include restricting the speed of the vehicle, controlling the direction of the vehicle...

Driver alert intervention may include use of sprays designed to stimulate the driver, vibrating the steering wheel, seat belt or floor area in the vicinity of the driver, an audible alarm and/or use of bright cabin lights. The driver can also be alerted by opening the driver window and/or other effective alerting methods as may be applicable to each individual driver.

Drivers of other vehicles may also be alerted by means of flashing hazard lights and/or sounding of a siren and/or by communication means (GSM, ...) to provide early warning indication that a driver is distracted or drowsy.

Because a low vigilance state could lead to unsafe driving conditions, the processing unit 30 shown in Figure 1 is designed to detect the drowsiness state and the distraction level of the driver.

To reduce in practice such a method of detection, the processing unit 30 is designed to implement first a learning phase (during which it adjusts its parameters of detection as a function of the driver's behavior, thanks to the first and second sensors 10, 20), then an action phase (during which it detects the drowsiness state and the distraction level of the driver thanks solely to the first sensors 10).

For the clarity of the description, we will start by describing the action phase. The learning phase will be described after.

During the action phase, the processing unit 30 implements three main steps that consist in:

a) measuring a variable $\varepsilon$, $\pi$, $\Omega$ relative to the contact or to the pressure exerted by the hands of the driver on the steering wheel 1, thanks to the first sensors 10,

b) comparing said variable $\varepsilon$, $\pi$, $\Omega$ with a first threshold $\varepsilon_1$, $\pi_1$, $\Omega_1$, and

c) deducing from the result of said comparison a first value $VS_1$ of the vigilance state VS of the driver.

During step a), the variables measured by the first sensors are the hand pressure $\pi$ exerted by the hand of the driver on the steering wheel 1, the gripping force $\varepsilon$ exerted by the fingers of the driver on the steering wheel 1 and the contact area $\Omega$ between the hand(s) of the driver and the steering wheel 1.

During step b), the instant value of each variable

$\epsilon$, $\pi$, $\Omega$ is compared with a first threshold $\epsilon_1$, $\pi_1$, $\Omega_1$, whose value is determined during the learning phase and remains constant during the action phase.

Step c) is implemented according to the following method.

First, the processing unit 30 determines if the value of the hand pressure $\pi$ has been below the corresponding first threshold $\pi_1$ for a duration that is greater than a predetermined amount of time $\Delta T_1$ (see Fig.2A).

If it is, a value of 2 is assigned to a first drowsiness level $VS_{DL1}$. Otherwise, a value of 1 is assigned to said first drowsiness level $VS_{DL1}$ (see Fig.2B). The value of the first drowsiness level $VS_{DL1}$, remains equal to 2 as long as the hand pressure $\pi$ remains below a second threshold $\pi_2$. Then, it is reduced to 1.

This second threshold $\pi_2$, whose value is greater than the one of the first threshold $\pi_1$, may be predetermined and stored in the ROM memory of the processing unit 30. Here, it is determined during the learning phase, as it will be explained below.

Then, the processing unit 30 determines if the value of the gripping force $\epsilon$ has been below the corresponding first threshold $\epsilon_1$ for a duration that is greater than a predetermined amount of time $\Delta T_2$ (see Fig.2A).

If it is, a value of 2 is assigned to a second drowsiness level $VS_{DL2}$. Else, a value of 1 is assigned to said second drowsiness level $VS_{DL2}$ (see Fig.2B). The value of the second drowsiness level $VS_{DL2}$ remains equal to 2 as long as the gripping force $\epsilon$ remains below a second threshold $\epsilon_2$. Then, it is reduced to 1.

This second threshold $\epsilon_2$ may be predetermined and stored in the ROM memory of the processing unit 30. Here, it is determined during the learning phase, as it will be explained below.

Finally, the processing unit 30 determines if the value of the contact area $\Omega$ has been below the corresponding first threshold $\Omega_1$ for a duration that is greater than a predetermined amount of time $\Delta T_3$ (see Fig.2A).

If it is, a value of 2 is assigned to a distraction level $VS_{DL3}$. Else, a value of 1 is assigned to said distraction level $VS_{DL3}$ (see Fig.2B). After it has been set to 2, the value of the distraction level $VS_{DL3}$ remains equal to 2 as long as the contact area $\Omega$ remains below a second threshold $\Omega_2$. Then, it is reduced to 1.

This second threshold $\Omega_2$ may be predetermined and stored in the ROM memory of the processing unit 30. Here, it is determined during the learning phase, as it will be explained below.

In the present embodiment, the predetermined amounts of time $\Delta T1$, $\Delta T_2$, $\Delta T_3$ are equal and are chosen between 3 seconds and 10 seconds.

Then, the processing unit 30 calculates the first value $VS_1$ of the vigilance state VS.

Here, this first value $VS_1$ is equal to the multiplication of the first drowsiness level $VS_{DL1}$, by the second drowsiness level $VS_{DL2}$ and by the distraction level $VS_{DL3}$:

$$VS_1 = VS_{DL1} \cdot VS_{DL2} \cdot VS_{DL3}$$

It is understood that this first value $VS_1$ will be even greater when the vigilance state of the driver will be low. This first value $VS_1$ is comprised between 1 and 8.

That is why, upon detecting a vigilance state which is strictly higher than an acceptable threshold (for instance 2), the processing unit 30 may alert the driver of the vehicle and/or the drivers of other vehicles, and may intervene in the control of the vehicle, thanks to the actuator means 40.

We now may detail the learning phase, which consists in determining the value of the first threshold $\epsilon_1$, $\pi_1$, $\Omega_1$, and of the second threshold $\epsilon_2$, $\pi_2$, $\Omega_2$, thanks to the parameters determined by the first sensors 10 and by the video camera 20.

Indeed, because the habits of the drivers while driving vehicles are different, the invention suggests to determine the thresholds value in such a manner that these thresholds take into account these different habits.

This learning phase may be implemented after each starting of the motor of the vehicle, or each time that the video camera 20 detects a new driver, or when the driver selects an operation of storing a new driver's profile in the memory of the processing unit 30.

The learning phase includes five main steps.

During a first step S1), the processing unit 30 sets each first threshold $\epsilon_1$, $\pi_1$, $\Omega_1$ to a predetermined default value $\epsilon_0$, $\pi_0$, $\Omega_0$, according to the following mathematic equalities:

$$\epsilon_1 = \epsilon_0$$

$$\pi_1 = \pi_0$$

$$\Omega_1 = \Omega_0$$

The predetermined default values $\epsilon_0$, $\pi_0$, $\Omega_0$ are stored in the ROM memory of the processing unit 30 and are equals to average values observed on a test population used for preliminary measures.

During a second step S2), the processing unit 30 compares each instant variable $\epsilon$, $\pi$, $\Omega$ (measured by the first sensors 10) with the associated first threshold $\epsilon_1$, $\pi_1$, $\Omega_1$.

Then, during a third step S3) that is identical to the method explained here-above in connection with the action phase, the processing unit 30 deduces from said comparison the first value $VS_1$ of the vigilance state VS. This first value is considered as a preliminary value $VS_0$.

During a fourth step S4), the processing unit determines a second value $VS_2$ of the vigilance state VS, thanks to the signal received from the second sensor 20.

In the represented embodiment, because this second sensor is a video camera 20, the second value $VS_2$ of the vigilance state VS is determined on the basis of the acquired images of the face of the driver. For this purpose, the images are processed to determine the movements of the lids of the driver and to deduce therefrom rates or degrees of eye closure. This rate or degrees of eye closure may be used to determine the second value $VS_2$ of the vigilance state VS.

Here, the second value $VS_2$ of the vigilance state VS obtained is scored on a scale of 1 to 8, 1/8 corresponding to a high degree of vigilance (when the driver lids closes at a normal frequency) and 8/8 corresponding to a low degree of vigilance (when the driver lids never closes or remains closed during long periods).

During a fifth step S5, the processing unit 30 adjusts the value of each first threshold $\varepsilon_1$, $\pi_1$, $\Omega_1$ as a function of the difference $\delta$ between said preliminary value $VS_0$ and said second value $VS_2$:

$$\delta = VS_0 - VS_2$$

Indeed, the absolute value $|\delta|$ of this difference $\delta$ corresponds to a correctness variable which indicates whether the instant value of each first threshold is able to detect the vigilance state of the driver with a high acuity or not.

To determine if the value of each first threshold is correct, the results of the processes used to determine the preliminary value $VS_0$ (thanks to the first sensors) and the second value $VS_2$ (thanks to the camera video 20) are compared.

To do it, the absolute value $|\delta|$ of the difference $\delta$ is compared to a correctness level $\xi$ (which is equal to 2 in this embodiment). This correctness level $\xi$ is defined as a threshold for which it is decided to stop the learning phase.

As a general rule, the comparison between the absolute value $|\delta|$ of the difference $\delta$ and the correctness level $\xi$ may allow the algorithm to check if the percentage of correctly predicated outputs out of the total number of predictions made by the algorithm is sufficient. When this percentage reaches a value above a predefined threshold ($\xi$), the algorithm will detect that it is started to work correctly and there is no need for more learning.

In the simplified described embodiment of the invention, the value of each first threshold $\varepsilon_1$, $\pi_1$, $\Omega_1$ is increased of a predetermined percentage (for instance 1%) if the difference $\delta$ is positive and is greater than 2. Each first threshold $\varepsilon_1$, $\pi_1$, $\Omega_1$ is decreased of a predetermined percentage (for instance 1%) if the difference $\delta$ is negative and is lower than -2. Otherwise, the value of each first threshold $\varepsilon_1$, $\pi_1$, $\Omega_1$ remains constant.

In this last case, because the difference $\delta$ is comprised between -2 and 2, the processing unit 30 switches to the action phase.

Otherwise, steps S1) to S5) are repeated until the absolute value $|\delta|$ of the difference $\delta$ becomes lower than or equal to 2.

Before switching to the action phase, the processing unit 30 calculates the second thresholds $\varepsilon_2$, $\pi_2$, $\Omega_2$. Here, the second thresholds $\varepsilon_2$, $\pi_2$, $\Omega_2$ are calculated thanks to the following equations:

$$\varepsilon_2 = \alpha \,.\, \varepsilon_1$$

$$\pi_2 = \alpha \,.\, \pi_1$$

$$\Omega_2 = \alpha \,.\, \Omega_1$$

In these equations, $\alpha$ is a predetermined constant stored in the ROM memory of the processing unit, that is strictly greater than 1 and that is for example equal to 1,1.

Last, first thresholds $\varepsilon_1$, $\pi_1$, $\Omega_1$ may be used as a source for learning about the driver's driving behaviors.

Indeed, low first thresholds $\varepsilon_1$, $\pi_1$, $\Omega_1$ indicate that the diver is used to have low or no contact with the steering wheel 1 for long periods, which could mean risky driving behaviors.

On the contrary, high first thresholds $\varepsilon_1$, $\pi_1$, $\Omega_1$ indicate that the diver is mentally stressed or overloaded.

**Claims**

1. A method for determining a vigilance state (VS) of a driver of a vehicle that comprises a steering wheel (1) including at least a first sensor (10) adapted to detect a contact or a pressure exerted by the hand of said driver on said steering wheel (1), said method including steps of:

   - measuring a variable ($\varepsilon$, $\pi$, $\Omega$) relative to the contact or to the pressure exerted by the hand of the driver on the steering wheel (1), thanks to said first sensor (10),
   - comparing said variable ($\varepsilon$, $\pi$, $\Omega$) with a first threshold ($\varepsilon_1$, $\pi_1$, $\Omega_1$), and
   - deducing from the result of said comparison a first value ($VS_1$) of the vigilance state (VS) of the driver,

   **characterized in that**, the vehicle comprising at least a second sensor (20) adapted to measure a parameter used to determine a second value ($VS_2$) of the vigilance state (VS) of the driver, said first threshold ($\varepsilon_1$, $\pi_1$, $\Omega_1$) is determined on the basis of said second value ($VS_2$).

2. The method according to claim 1, wherein the determination of said first threshold ($\varepsilon_1$, $\pi_1$, $\Omega_1$) is implemented during a learning phase that is prior to an action phase during which said first value ($VS_1$) is deduced from the result of said comparison.

3. The method according to claim 2, wherein the learning phase includes steps of:

   S1) setting said first threshold ($\varepsilon_1$, $\pi_1$, $\Omega_1$) to a predetermined default value,
   S2) comparing said variable ($\varepsilon$, $\pi$, $\Omega$) with the default value of the first threshold ($\varepsilon_1$, $\pi_1$, $\Omega_1$),
   S3) deducing from the result of step S2) a preliminary value ($VS_0$) of said vigilance state (VS),
   S4) acquiring the second value ($VS_2$) of said vigilance state (VS), and
   S5) adjusting the value of said first threshold ($\varepsilon_1$, $\pi_1$, $\Omega_1$) depending on said preliminary value ($VS_0$) and said second value ($VS_2$).

4. The method according to claim 3, wherein the learning phase also includes steps of:

   S6) determining a correctness variable ($|\delta|$) depending on said preliminary value ($VS_0$) and said second value ($VS_2$), and
   S7) if said correctness variable ($|\delta|$) is above a correctness level ($\xi$), switching to said action phase, else, repeating steps S1) to S5).

5. The method according to anyone of claims 1 to 4, wherein, said second sensor (20) being adapted to acquire images of the face of the driver, said first threshold ($\varepsilon_1$, $\pi_1$, $\Omega_1$) is determined on the basis of the movements of the eyelids of the driver detected on the acquired images.

6. The method according to anyone of claims 1 to 5, wherein the variable ($\pi$) measured by said first sensor (10) is a hand pressure exerted by the hand of the driver on the steering wheel (1), and the first value ($VS_1$) of said vigilance state (VS) is equal to or deduced from a first drowsiness level ($VS_{DL1}$) determined on the basis of the result of the comparison between said variable ($\pi$) and said first threshold ($\pi_1$).

7. The method according to anyone of claims 1 to 6, wherein the variable ($\varepsilon$) measured by said first sensor (10) is a gripping force exerted by the driver on the steering wheel (1), and the first value ($VS_1$) of said vigilance state (VS) is equal to or deduced from a second drowsiness level ($VS_{DL2}$) determined on the basis of the result of the comparison between said variable ($\varepsilon$) and said first threshold ($\varepsilon_1$).

8. The method according to claim 7, wherein the first value ($VS_1$) of said vigilance state (VS) is equal to or deduced from the multiplication of said first drowsiness level ($VS_{DL1}$) by said second drowsiness level ($VS_{DL2}$).

9. The method according to anyone of claims 1 to 8, wherein the variable ($\Omega$) measured by said first sensor (10) is a contact area between the hand of the driver and the steering wheel (1), and the first value ($VS_1$) of said vigilance state (VS) is equal to or deduced from a distraction level ($VS_{DL3}$) determined on the basis of the result of the comparison between said variable ($\Omega$) and said first threshold ($\Omega_1$).

10. The method according to anyone of claims 1 to 9, wherein at said step of deduction, the first value ($VS_1$) of said vigilance state (VS) is reduced if said variable ($\varepsilon$, $\pi$, $\Omega$) remains below said first threshold ($\varepsilon_1$, $\pi_1$, $\Omega_1$) during a predetermined amount of time ($\Delta T$).

11. The method according to claim 10, wherein at said step of deduction, the first value ($VS_1$) of said vigilance state (VS), after it has been reduced, is increased if said variable ($\varepsilon$, $\pi$, $\Omega$) rises above a second threshold ($\varepsilon_2$, $\pi_2$, $\Omega_2$) that is distinct from said first threshold ($\varepsilon_1$, $\pi_1$, $\Omega_1$).

12. The method according to anyone of claims 1 to 11, comprising a last step of determining a parameter relative to the behavior for said driver, on the basis of said first threshold ($\varepsilon_1$, $\pi_1$, $\Omega_1$).

# Fig.1

# Fig.2A

# Fig.2B

Europäisches
Patentamt

European
Patent Office

Office européen
des brevets

# EUROPEAN SEARCH REPORT

Application Number

EP 16 16 1159

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | US 2009/209829 A1 (YANAGIDAIRA MASATOSHI [JP] ET AL) 20 August 2009 (2009-08-20)<br>* paragraphs [0001], [0056], [0067], [0070], [0071]; claims; figures *<br>----- | 1,2,5,<br>10,12<br>6,7,9<br>3,4,8,11 | INV.<br>A61B5/16<br>B60K35/00<br>B60W40/08<br>G01C21/36 |
| A | US 9 248 839 B1 (TAN ADRIAN [US])<br>2 February 2016 (2016-02-02)<br>* column 9, lines 33-67; claims; figures *<br>----- | 1-12 | |
| A | EP 1 548 678 A1 (DAIMLER CHRYSLER AG [DE])<br>29 June 2005 (2005-06-29)<br>* abstract; claims; figures *<br>----- | 1-12 | |
| Y,D | US 2011/246028 A1 (LISSEMAN JASON [US] ET AL) 6 October 2011 (2011-10-06)<br>* paragraphs [0006], [0007]; claims; figures *<br>----- | 6,7,9 | |
| A | US 2016/009175 A1 (MCNEW JOHN-MICHAEL [US]) 14 January 2016 (2016-01-14)<br>* paragraphs [0071] - [0073]; claims; figures *<br>----- | 1-12 | TECHNICAL FIELDS SEARCHED (IPC)<br><br>A61B<br>B60K<br>B60W<br>G01C |

The present search report has been drawn up for all claims

| Place of search | Date of completion of the search | Examiner |
|---|---|---|
| The Hague | 13 September 2016 | Ducher, Alban |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 16 16 1159

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2016

| Patent document cited in search report | | Publication date | Patent family member(s) | | Publication date |
|---|---|---|---|---|---|
| US 2009209829 | A1 | 20-08-2009 | JP | 4739407 B2 | 03-08-2011 |
| | | | US | 2009209829 A1 | 20-08-2009 |
| | | | WO | 2007111223 A1 | 04-10-2007 |
| US 9248839 | B1 | 02-02-2016 | US | 9248839 B1 | 02-02-2016 |
| | | | US | 2016107654 A1 | 21-04-2016 |
| EP 1548678 | A1 | 29-06-2005 | DE | 10355221 A1 | 23-06-2005 |
| | | | EP | 1548678 A1 | 29-06-2005 |
| | | | EP | 1687788 A1 | 09-08-2006 |
| | | | ES | 2300844 T3 | 16-06-2008 |
| | | | ES | 2321293 T3 | 04-06-2009 |
| | | | JP | 4825132 B2 | 30-11-2011 |
| | | | JP | 2005158077 A | 16-06-2005 |
| | | | JP | 2007514467 A | 07-06-2007 |
| | | | US | 2005128092 A1 | 16-06-2005 |
| | | | US | 2009115589 A1 | 07-05-2009 |
| | | | WO | 2005059857 A1 | 30-06-2005 |
| US 2011246028 | A1 | 06-10-2011 | DE | 102011006713 A1 | 15-12-2011 |
| | | | JP | 2011219085 A | 04-11-2011 |
| | | | JP | 2016052886 A | 14-04-2016 |
| | | | US | 2011246028 A1 | 06-10-2011 |
| US 2016009175 | A1 | 14-01-2016 | DE | 102015110348 A1 | 14-01-2016 |
| | | | US | 2016009175 A1 | 14-01-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20110246028 A